# EUROPEAN PATENT APPLICATION

(11) **EP 0 875 512 A2**
(43) Date of publication of application: **04.11.1998**
(21) Application number: 98500085.0
(22) Date of filing: 07.04.1998
(51) Int. Cl.: C07D 403/06, A61K 31/495

(54) **New naphthylpiperazine derivatives with antipsychotic activity**

(30) Priority: 16.04.1997 ES 9700812
(71) Applicant: FABRICA ESPANOLA DE PRODUCTOS QUIMICOS Y FARMACEUTICOS, S.A. (FAES), E-48940 Lejona-Lamiaco (Vizcaya) (ES)
(72) Inventor: Orjales Venero, Aurelio, 48990 Neguri (Vizcaya) (ES); Garcia Dominguez, Neftali, 48990 Getxo (Vizcaya) (ES)
(74) Representative: Isern-Cuyas, Maria Luisa

(57) **Abstract**

A description is given of new naphthylpiperazine derivatives of general formula (I) 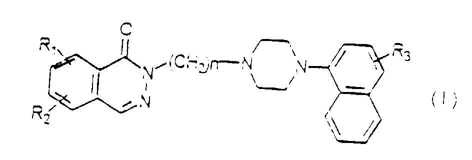 in which R₁ and R_{2,} equal to or different from each other, can be hydrogen, a short chain alkyl group, halogen, a nitro group, an amino group, an acylamino group and a short chain alkoxy group; n can take values between 2 and 5; and R₃ can be a methoxyl radical, a fluorine and a hydrogen atom, and their addition salts with pharmaceutically acceptable acids.

The compounds present affinity for serotonin 5HT_{1A} and 5HT₂ receptors and for dopamine D₂ receptors, and can be useful as antipsychotics.

Their preparation is also described.

## Description

### Object of the Invention

The present invention relates to a number of new naphthylpiperazine derivatives and their addition salts. as well as their preparation process. The new compounds present serotonergic and central dopaminergic activity and are useful as antipsychotics.

### Background of the Invention

The most widely accepted theory explaining the biochemical bases of schizophrenia holds that the dopaminergic activity in the mesolimb system of the brain is increased and, accordingly, the pharmacological power of classic antipsychotics is correlated with their affinity for D₂ receptors (Science 1976, 1982,481-483). It has further been proposed (J. Pharm. Exp. Ther., 1989, 251, 238-246) that a high affinity for 5HT₂ receptors in atypical antipsychotics would be responsible for the beneficial effects of the pharmacological profile of such atypical antipsychotics. It has moreover been found (J. Neurol. Transm., 1983, 57, 255) that 5HT_{1A} antagonists are capable of inverting haloperidol-induced catalepsy. Thus, compounds with affinity for 5HT_{1A}, 5HT₂ and D₂ receptors behave as atypical antipsychotics (Advances in Med. Chem., 3, 1995, 1-55).

European Patent EP 0329168 describes a number of 1,4-disubstituted piperazines with psychotropic activity in which the 1-substituent is a bicyclic heterocycle which incorporates an amide or imide function and the 4-substituent is a heterocycle.

US 4,983,606 discloses a number of compounds with anti-platelet aggregating, anti-vasospastic and anti-proliferative activities which carry the phthalazinone group bonded to a phenyl(benzoyl)piperazine or phenyl(benzoyl)piperidine fragment through a carbon chain.

J. Med. Chem. 1994, 37, 1060-1062 describes a number of new arylpiperazines having a high affinity for D₂, D₃, 5HT_{1A} and α_{1A} receptors, providing them with interesting antipsychotic properties, moreover presenting a low potential for causing extrapyramidal effects. Finally, Patent WO 93/16073 describes a number of arylpiperidines and arylpiperazines with D₂ and 5HT₂ receptor antagonist properties, useful in the treatment of psychosis.

The present invention relates to a number of new naphthylpiperazine derivatives of formula (I) and their addition salts with pharmaceutically acceptable acids, 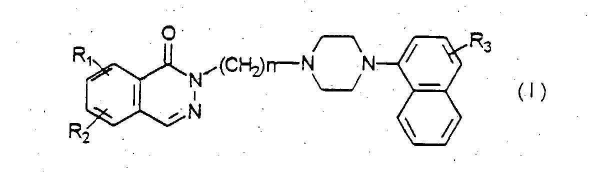 in which R₁ and R₂ (equal to or different from each other) can be hydrogen, a short chain alkyl group, halogen,- a nitro group, an amino group, an acylamino group and a short chain alkoxy group; n can take values between 2 and 5, and R₃ can be a methoxyl radical, a hydrogen atom, and a fluorine atom.

The compounds described herein are essentially different from those referred to in the above-mentioned publications, due to the presence of the naphthylpiperazine fragment, which pharmacologically characterises them and provides them with a great affinity for 5HT_{1A} and 5HT₂ receptors. These new compounds also have a great affinity for the D₂ receptor and are therefore useful as atypical antipsychotics.

### Description of the Invention

The preparation of compounds of formula (I) can be carried out along various synthetic routes, using conventional methods:

### a) Condensation of a compound of formula (II)

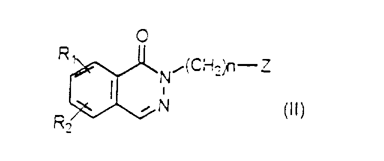

in which R₁ and R₂ have the meaning specified for formula (I) and Z is chlorine or bromine, with a piperazine of formula (III)

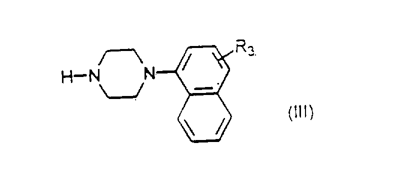

The reaction is carried out in an inert solvent, in the presence of a base, and at a temperature ranging between 20°C and the boiling point of the reaction mixture.

Examples of used solvents are dichloromethane, chloroform, acetonitrile, dimethylformamide and tetrahydrofurane.

Used bases can be an alkaline carbonate or bicarbonate, such as K₂CO₃, KHCO₃, Na₂CO₃, Na₂HCO₃ or a tertiary amine such as pyridine or triethylamine.

The reaction rate may be increased by adding catalytic amounts of an alkaline iodide, such as KI to the reaction mixture.

Piperazines of formula (III) are prepared by reacting bis (2-chloroethyl)amine with the corresponding naphthylamine. Compounds of general formula (II) used as starting products at the previous condensation stage are prepared by reacting a compound of general formula (IV) 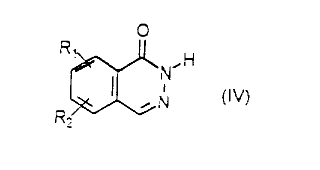 in which R₁ and R₂ have been previously defined, with an alkyl dihalide in the presence of a strong base, such as NaH in an aprotic solvent such as dimethylformamide or tetrahydrofurane, or solid potassium hydroxide in dimethylformamide, at a temperature ranging between room temperature and 100°C. Compounds of general formula (IV) are prepared by reacting suitable 2-formylbenzoic acids with hydrazine hydrate.

Alternately, compounds of general formula (II) can be prepared by substitution of the hydroxyl group in compounds of formula (V) by chlorine or bromine.

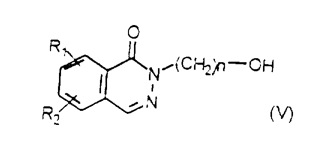

The substitution reaction can be carried out by treatment with hydrogen chloride, hydrogen bromide, or by treatment with organic or inorganic acid halides, such as thionyl chloride, oxalyl chloride, in an inert solvent such as chloroform, dichloromethane, or toluene, or using the acid chloride as a solvent, at a temperature ranging between 0°C and the boiling point of the reaction mixture.

Compounds of general formula (V) can in turn be prepared as specified in Chimie Thérapeutique, 1967, 2, 250-253.

As noted hereinbefore, the new compounds of general formula (I) show SNC activity, specifically at the 5HT_{1A}, 5HT₂ and D₂ receptor levels.

### PHARMACOLOGICAL RECEPTOR BINDING STUDIES.

Binding studies have been carried out to ascertain the affinity of the compounds for 5HT_{1A}, 5HT₂ and D₂ receptors.

### 5HT_{1A} RECEPTOR

Cerebral cortex taken from both male and female Wistar rats was homogenised in saccharose buffer 0.32 M (1:10 g/mL) and centrifuged at 900 *g* (10 min, 4°C). The supernatant was collected and centrifuged at 48000 g (25 min, 4°C). The sediment thus obtained was resuspended in cold TRIS buffer 50 mM (pH 7.5, 1:10, g/mL), homogenised, incubated at 37°C for 15 min and centrifuged again at 48000 *g* (25 min. 4°C). The final sediment was re-suspended in cold SNAYDER buffer (1:4, g/mL), homogenised and kept at -70°C in 5 mL containers.

For the displacement trial, 100 µL of radioligand (2 nM, final conc.), 100 µL of the different tested concentrations of the displacing product and 750 µL of a suspension of membranes 1:32 in SNAYDER with pargilin were used. The volume was topped up to I mL with 50 µL of SNAYDER. Serotonin (5HT) 10 µM was used to define the non-specific binding.

### 5HT₂ RECEPTOR

Prefrontal cortex taken from both male and female Wistar rats was homogenised in saccharose buffer 0.25 M (1:10 g/mL) and centrifuged at 1080 *g* (10 min, 4°C). The supernatant was set aside and the pellet re-suspended in the same buffer (1:5, g/mL) and centrifuged again under the same conditions. The mixture of both supernatants was topped up to 1:40 (g/mL) with TRIS buffer 50 mM (pH 7.7) and centrifuged at 35000 *g* (10 min, 4°C). The sediment thus obtained was re-suspended in cold TRIS buffer (1:40, g/mL) and centrifuged again at 35000 *g* (10 min, 4°C). The final sediment was re-suspended in cold TRIS buffer (1:10, g/mL), homogenised and kept at -70°C in 5 mL containers.

For the displacement trial, 100 µL of radioligand (0.5-1 nM, final concentration), 100 µL of the different tested concentrations of the displacing product and 750 µL of a suspension of membranes 1:50 (0.54 mg/mL, 15 mg fresh tissue) in TRIS were used. The volume was topped up to 1 mL with 50 µL of TRIS/10% ethanol. Metisergide 1 µM was used to define the non-specific binding. The displacer, radioligand and Metisergide dilutions were all made with TRIS buffer with 10% ethanol (v/v).

### D₂ RECEPTOR

Corpus striatum taken from both male and female Wistar rats was homogenised in TRIS buffer 50 mM (pH 7.7, 1:50 g/mL) and centrifuged at 47800 *g* (10 min, 4°C). The supernatant was eliminated and the pellet re-suspended in the same buffer (1:50, g/mL), incubated at 37°C for 10 min and centrifuged again under the same conditions. The final sediment thus obtained was re-suspended in cold TRIS buffer 50 mM (pH 7.4, 1:10, g/mL) containing NaCl 120 mM + KCl 5 mM + CaCl₂ 2 mM + MgCl₂ 1 mM + ascorbic acid 0.01% g/mL, and kept at -70°C in 2.5 mL aliquots. Membrane dilutions (1:100-1:300) were subsequently carried out and the amount of proteins was assessed by the Lowry method.

For the displacement test, 100 µL of radioligand (1-2 nM, final conc.), 100 µL of the different tested concentrations of the displacing product and 750 µL of a suspension of membranes 1:150 (0.39-0.13 protein mg/mL) in the previous (saline) TRIS + 10 µM of pargilin were used. The volume was topped up to 1 mL with 50 µL of the previous TRIS. Butachlamol 1 µM was used to define the non-specific binding, which was added (100 µL) to the blank series. The displacer, radioligand and Butachlamol dilutions were all made with TRIS (saline) buffer + pargilin. The samples were incubated for 60 min at 25°C.

The products described in the present invention have shown a high (nanomolar range) affinity for the three tested receptors, which renders them potentially useful as antipsychotics.

The following examples provide further details of the invention, which is not howsoever limited to such examples.

### Example 1.

### 2-(4-Bromobutyl)-1(2H)-phthalazinone.

Small portions (2.5 g, 60 mmol) of a dispersion of 60% NaH in mineral oil were added over a solution of 1(2H)-phthalazinone (7.3 g, 50 mmol) in dimethylformamide (100 mL) cooled to 0°C. The reaction mixture was left to reach room temperature and kept stirred for an hour. 1,4-dibromobutane (18 mL, 150 mmol) was then added at a time and the reaction mixture was stirred, at room temperature, for 4 hours. The reaction mixture was poured over cnished ice, extracted with ethyl ether (twice), and the organic extracts were dried over anhydrous Na₂SO₄ and concentrated to dryness. The excess dibromobutane was eliminated by distillation and the obtained residue was purified by flash chromatography (CH₂Cl₂), yielding an oil (11.6 g, yield: 83%) identified on the basis of its spectroscopic data as the title product

### Example 2.

### 2-[4-(4-naphthylpiperazine-1-yl)-butyl]-1(2H)-phthalazinone.

A mixture of 2-(4-bromobutyl)-1(2H)-phthalazinone (1.7 g, 6 mmol), 1-naphthylpiperazine (1.3 g, 6 mmol), K₂CO₃ (0.84 g, 6 mmol), and KI (10 mg) in acetonitrile (50 mL) was refluxed for 4 hours. When heating was over, the solvent was eliminated at reduced pressure and the residue distributed between dichloromethane and water; the aqueous phase was extracted with dichloromethane (twice). The organic extracts were collected, dried over anhydrous Na₂SO₄ and concentrated to dryness. The obtained residue was purified by flash column (CH₂Cl₂/MeOH 97:3), yielding 2-[4-(4-naphthylpiperazine-1-yl)-butyl]-1(2H)-phthalazinone (1.7 g). Treatment with hydrochloric ethanol yielded a white solid, which was re-crystallised in methanol, yielding the monohydrochloride of the title compound, presenting a M.P. higher than 260°C.

### Example 3.

### 2-(2-hydroxyethyl)-6,7-dimethoxy-1(2H)-phthalazinone.

2-hydroxyethylhydracine (4.2 g, 55 mmol) was added to a solution of 6-formyl-3,4-dimethoxybenzoic acid (10.5 g, 50 mmol) in ethanol (100 mL). The reaction mixture was refluxed for 3 hours, after which the solvent was eliminated at reduced pressure. The resultant residue was treated with ethyl ether to yield a white solid which was collected by filtration, yielding 2-(2-hydroxyethyl)-6,7-dimethoxy-1(2H)-phthalazinone (9.2 g, yield: 76%). M.P. = 176-179°C.

### Example 4.

### 2-(2-chloroethyl)1-6,7-dimethoxy-1(2H)-phthalazinone.

Thionyl chloride (15 mmol) was added to a solution of 2-[2-(hydroxyethyl)]-6,7-dimethoxy-1(2H)-phthalazinone (2.5 g, 10 mmol) in chloroform (50 mL) and the mixture was kept stirred at room temperature overnight. The solvent was eliminated at reduced pressure, and the obtained solid was treated with hexane, filtered and purified by flash chromatography (CH₂Cl₂), yielding 2-(2-chloroethyl)-6,7-dimethoxy-1(2H)-phthalazinone (2.2 g, yield: 81%). M.P. 178-181°C.

### Example 5.

### 2-[2-(4-naphthylpiperazine-1-yl)-ethyl-6,7-dimethoxy-1(2H)-phthalazinone.

A mixture of 2-(2-chloroethyl)-6,7-dimethoxy-1(2H)-phthalazinone (1.6 g, 6 mmol), 1 -naphthylpiperazine (1.3 g, 6 mmol), K₂CO₃ (0.84 g, 6 mmol) and KI (10 mg) in acetonitrile (50 mL) was refluxed for 7 hours. When heating was over, the solvent was eliminated at reduced pressure and distributed between dichloromethane and water, and the aqueous phase was extracted with dichloromethane (twice). The organic extracts were collected. dried over anhydrous Na₂SO₄ and concentrated to dryness. The obtained residue was purified by flash chromatography (CH₂Cl₂/MeOH 97:3) yielding 2-[2-(4-naphthylpiperazine-1-yl)-ethyl]-6,7-dimethoxy-1(2H)-phthalazinone (1.5 g). Treatment with hydrochloric ethanol yielded a white solid, which was re-crystallised in methanol, yielding the monohydrochlorate of the title compound, presenting a M.P. higher than 260°C.

## Claims

1. New naphthylpiperazine derivatives of general formula (I) 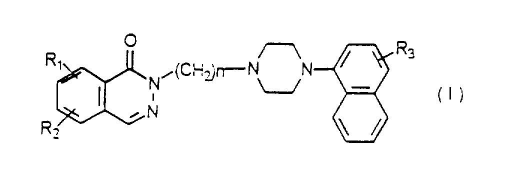 in which R₁ and R₂, equal to or different from each other, are hydrogen, a short chain alkyl group, halogen, a nitro group, an amino group, an acylamino group and a short chain alkoxy group; n can take values between 2 and 5; and R₃ can be a methoxyl radical, a fluorine and a hydrogen atom.

2. New naphthylpiperazine derivatives, in accordance with claim 1, characterised by comprising a compound consisting of 2-[4-(4-naphthylpiperazine-1-yl)-butyl]-1(2H)-phthalazinone.

3. New naphthylpiperazine derivatives, in accordance with claim 1, characterised by comprising a compound consisting of 2-[3-(4-naphthylpiperazine-1-yl)-propyl]-1(2H)-phthalazinone.

4. New naphthylpiperazine derivatives, in accordance with claim 1, characterised by comprising a compound consisting of 2-[2-(4-naphthylpiperazine-1-yl)-ethyl]-1(2H)-phthalazinone.

5. New naphthylpiperazine derivatives, in accordance with claim 1, characterised by comprising a compound consisting of 2-[4-(4-naphthylpiperazine-1-yl)-butyl]-6,7-dimethoxy-1(2H)-phthalazinone.

6. New naphthylpiperazine derivatives, in accordance with claim 1, characterised by comprising a compound consisting of 2-[2-(4-naphthylpiperazine-1-yl)-ethyl]-6,7-dimethoxy-1(2H)-phthalazinone.

7. New naphthylpiperazine derivatives, in accordance with claim 1, characterised by comprising a compound consisting of 2-[4-(4-naphthylpiperazine-1-yl)-butyl]-7-nitro-1(2H)-phthalazinone.

8. New naphthylpiperazine derivatives, in accordance with claim 1, characterised by comprising a compound consisting of 2-[4-(4-naphthylpiperazine1-yl)-butyl]-7-amino-1(2H)-phthalazinone.

9. New naphthylpiperazine derivatives, in accordance with claim 1, characterised by comprising a compound consisting of 2-[4-(4-naphthylpiperazine-1-yl)-butyl]-7-acetylamino-1(2H)-phthalazinone.

10. New naphthylpiperazine derivatives, in accordance with claim 1, characterised by comprising a compound consisting of 2-[3-(4-naphthylpiperazine-1-yl)-propyl]-7-nitro-1(2H)-phthalazinone.

11. New naphthylpiperazine derivatives, in accordance with claim 1, characterised by comprising a compound consisting of 2-[4-(4-(7-methoxynaphthyl)-piperazine-1-yl)-butyl]-1(2H)-phthalazinone.

12. New naphthylpiperazine derivatives, in accordance with claim 1, characterised by comprising a compound consisting of 2-[3-(4-(7-methoxynaphthyl)-piperazine-1-yl)-propyl]-1(2H)-phthalazinone.

13. New naphthylpiperazine derivatives, in accordance with claim 1, characterised by comprising a compound consisting of 2-[4-(4-(7-methoxynaphthyl)-piperazine-1-yl)-butyl]-7-nitro-1(2H)-phthalazinone

14. New naphthylpiperazine derivatives, in accordance with claim 1, characterised by comprising a compound consisting of 2-[3-(4-(7-methoxynaphthyl)-piperazine-1-yl)-propyl]-7-nitro-1(2H)-phthalazinone.

15. New naphthylpiperazine derivatives, in accordance with claim 1, characterised by comprising a compound consisting of 2-[4-(4-(7-methoxynaphthyl)-piperazine-1-yl)-butyl]-6,7-dimethoxy-1(2H)-phthalazinone.

16. New naphthylpiperazine derivatives, in accordance with claims 1 to 15, characterised by comprising addition salts with pharmaceutically acceptable acids of the compounds of formula (I).

17. New naphthylpiperazine derivatives. in accordance with claims 1 to 15, characterised by the use of the compounds of formula (1) as antipsychotics.
